# EUROPEAN PATENT SPECIFICATION

(11) **EP 2 664 356 B1**
(45) Date of publication and mention of the grant of the patent: **30.09.2015**
(21) Application number: 12380025.2
(22) Date of filing: 18.05.2012
(51) Int. Cl.: A61N 1/36, A61N 1/04

(54) **System for neuropathic pain rehabilitation**
System zur Rehabilitierung neuropathischer Schmerzen
Système pour la réhabilitation de la douleur neuropathique

(43) Date of publication of application: 20.11.2013
(73) Proprietor: Fundació Institut Guttmann, 08916 Badalona (Barcelona) (ES)
(72) Inventor: Soler Fernandez, Maria Dolors, 08017 Barcelona (ES); Opisso Salleras, Eloy, 08301 Mataro (Barcelona) (ES)
(74) Representative: Torner Lasalle, Elisabet

(56) References cited:
- WO-A2-2007/138598
- WO-A2-2009/137683
- WO-A2-2011/123548
- US-A1- 2007 179 558
- US-A1- 2010 268 287
- US-A1- 2010 280 571
- US-B2- 7 856 264

## Description

### Field of the art

The present invention generally relates to a system that includes a transcranial direct current stimulation apparatus designed to be used by a patient at a place remote from a clinic location, i.e. allowing a home self-treatment of a neuropathic pain, under assistance of a computer device that guides and controls the correctness ad safety of the treatment, the system allowing a remote connection to a clinic station for further guidance or correction.

The invention also provides a method to use the system comprising providing, to a patient suffering from said neuropathic pain, a visual illusion to correct a mismatch between motor commands and sensory feedback, and more particularly to a method further comprising applying a direct cranial stimulation to said patient during at least part of the duration of said visual illusion.

The method of the invention is particularly applicable to patients suffering from spinal cord injuries.

### Prior State of the Art

Neuropathic pain affects ∼40% of patients following spinal cord injury (SCI) (Siddall et al., 2003; Widerström-Noga and Turk, 2003; Soler et al., 2007) and represents a highly disabling clinical condition. The injured spinal somatosensory circuitry is thought to generate aberrant nocioceptive impulses that are interpreted by the brain as pain (Yezierski, 2005). Thalamic integrative circuits may also act as generators and amplifiers of nocioceptive signals (Hains et al., 2005, Waxman and Haines, 2006). Sensory deafferentation after SCI induces profound and long-lasting reorganization of the cortical and subcortical sensory maps in the adult brain (Lotze et al., 2006; Kokotilo et al., 2009; Wrigley et al., 2009). Pathophysiological consequences of such cortical plasticity may underlie the development of phantom sensations and pain (Moore et al., 2000; Lotze et al., 2001; Wrigley et al., 2009; Soler et al., 2010). Strategies aimed at reversing or modulating the somatosensory neural reorganization after injury may be valuable alternative therapeutic approaches to neuropathic pain.

Examples of such approaches that have shown some promise for the treatment of neuropathic pain following SCI or limb amputation include the use of movement imagery (Gustin et al., 2008; Maclver et al., 2008), mirror therapy (Ramachandran and Hirstein, 1998; Giraux and Sirigu, 2003; Chan et al., 2007) or 'virtual' mirror therapy (Moseley, 2007). Longterm pain relief was achieved by repeated treatment sessions in which patients were given the visual illusion that they could move and use again their deafferented or missing limbs. Pain relief presumably involves correction of the incongruence between motor output and sensory feedback, and normalization of cortical somatosensory representation maps, induced by the visual input of movements of the paralysed/missing limbs (Ramachandran and Rogers-Ramachandran, 1996; Harris, 1999; Moseley, 2007). Mirror therapy may also contribute to modulate cortical and spinal excitability (Giraux and Sirigu, 2003; Stinear and Byblow, 2004; Garry et al., 2005; Funase et al., 2007; Sakamoto et al., 2009).

WO2009/137683 discloses a transcranial direct current stimulation apparatus and method that includes the features of the preamble of claim 1.

US patent application US 2013/0035734 of the same applicants refer to a method for treating neuropathic pain, designed to be implemented at a clinical site, comprising, provide to a patient suffering from said neuropathic pain, a visual illusion to correct a mismatch between motor commands and sensory feedback and applying a transcranial direct current stimulation to said patient during at least part of the duration of said visual illusion.

WO 2007/138598 discloses a method of rehabilitation management involving brain stimulation, comprising: providing a plurality of patients fitted with sensor; assigning task to said patients by a therapist; rehabilitating said patients not under the direct attention of said therapist and monitoring the rehabilitating using data acquired by the sensors during performance of the tasks.

US 7856264 disclose a system for patient Interactive neural stimulation comprising all the features of the preamble of claim 1 of this application. The system of US 7856264 is particularly intended for facilitate patient performance of activities in association with neural stimulation and/or chemical substance therapies to increase the efficacy and/or efficiency associated with such therapies.

The current invention provides a similar rehabilitation treatment as disclosed in cited US 2013/0035734 but implemented at home under self-control of the patient while remote complementary assistance is foreseen.

### Summary of the invention

Thereby the present invention proposes a system for neuropathic pain rehabilitation, including as already known in the art.
a transcranial direct current stimulation (tDCS) apparatus comprising:
- a cap sized to fit over a portion of a patient's head;
- two or more connecting points for electrodes operatively associated with an inner surface of the cap; and
- a power supply in electrical communication with the electrodes and with a control interface both said power supply and said control interface being sized to be worn by and transported by a patient in use,

The system as per this invention is characterized in that it further comprises:
a computer device on which a dedicated software runs and an associated control unit to provide a patient's self operation of said tDCS apparatus acting through said control interface during a neuropathic pain rehabilitation session initiated by said patient.;
at least a camera to capture at least a partial image of said patient;
a screen to present to said patient a combination of said partial patient's own image and a complementary image consisting in an animated digital image representative of a movement of a body injured part of the patient capable of generating a visual illusion suitable to correct mismatching between motor commands and sensory feedback, said combination of images being prepared or generated under control of said dedicated software and computer device.
means (at the control interface of the tDCS) for a checking of:
   - impedance of each of the connecting sections from each of the electrodes to the power source; and
   - polarity of each of the electrodes;
so that it can act as a security measure to confirm right positioning of the electrodes and feed current.

Each of the cited electrodes comprises a non invasive electrode pad and attachment member providing for the selective placement of the electrode at more than one possible position on the inner surface of the cap where a complementary attachment member and electrical connection exists.

Moreover the computer device comprises a net connection to a remote medical centre that can evaluate o supervise any of said patients sessions and provide to said patient complementary operating instructions.

Meanwhile the control interface includes an electronic circuit with a wireless communication unit allowing at least a communication with said computer device.

According to a preferred embodiment both of the control interface and the power supply are associated to the cap.

The control interface of the tDCS includes also means for further managing:
- duration and conditions of feeding of said electrical current that is a DC current;
- projection on the screen; and
- capture of the person's image or part thereof.

The invention in a second aspect provides a method for neuropathic pain rehabilitation by a tDCS treatment comprising following steps:
providing a patient with a tDCS apparatus at a location remote from a clinic location, said tDCS apparatus comprising as previously disclosed a cap sized to fit over a portion of a patient's head; two or more electrodes operatively associated with an inner surface of the cap and a power supply in electrical communication with the electrodes and with a control interface both said power supply and said interface being sized to be worn by and transported by a patient in use;
initiating tDCS treatment at a remote location away from the clinic location under a guidance of a computer device connected to said tDCS allowing said patient a self operation of said tDCS apparatus;
presenting in a screen to said patient a combination of a partial own image captured by a camera and a complementary image provided by an animated digital image representative of a movement of a body injured part of the patient capable of generating a visual illusion suitable to correct mismatching between motor commands and sensory feedback; and
controlling said combination of images being provided by said computer device in communication with said patient, the computer device also providing instructions about activity of the patient.

The method of this invention further includes guidance from a remote clinic station of any pain rehabilitation session by remote connection to said computer device.

The digital image can include augmented reality components to enhance perception and truth o plausibility of the represented movement of a body member o body part.

Other features of the invention will be apparent from the following description of one embodiment thereof.

### Brief description of the drawings

Fig. 1 is a perspective view showing the local scenario where the invention is implemented.
Fig. 2 is a diagram that discloses the architecture of the control interface and elements of the disclosed system related in order to implement the invention.

### Detailed description of embodiments of the invention

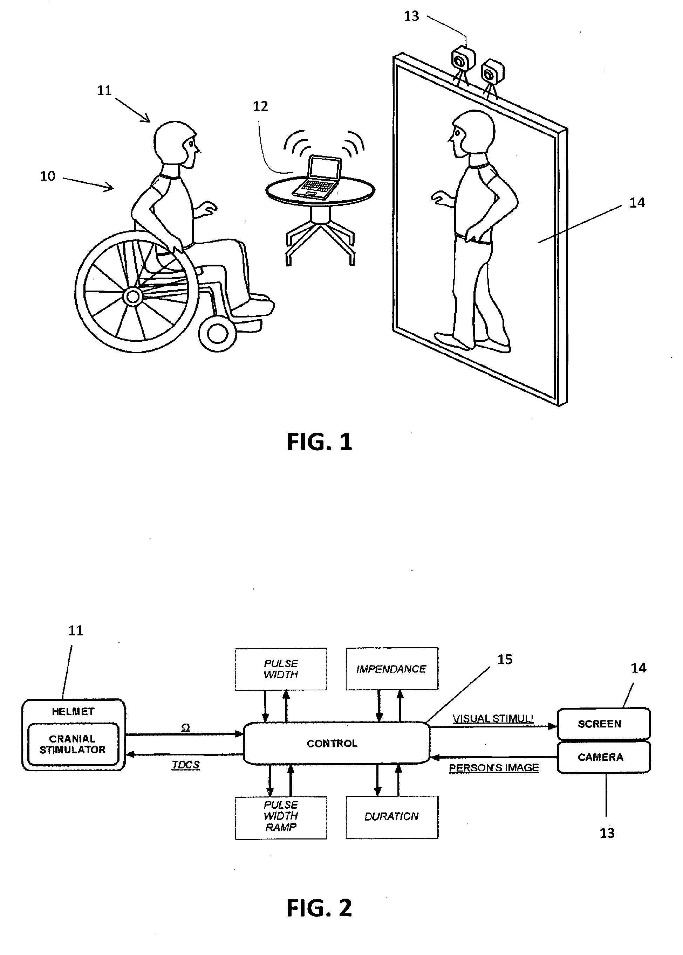

Fig. 1 discloses a patient 10, wearing a cap 11 for a transcranial direct current stimulation (tDCS), and a computer device 12 with a dedicated software related to an associated control unit (not illustrated) to provide to the patient a self operation of said tDCS , acting through a control interface (see Fig. 2) during a neuropathic pain rehabilitation session initiated by said patient.

As well known the cap 11 includes two or two or more connecting points for electrodes operatively associated with an inner surface of the cap, and a power supply in electrical communication with the electrodes and with a control interface 15 (Fig. 2) both said power supply and said control interface being sized to be worn by and transported by a patient in use.

Each of the electrodes comprises a non invasive electrode pad with and attachment member providing for the selective placement of the electrode at more than one possible position on the inner surface of the cap where a complementary attachment member and electrical connection exists. Each of said electrodes comprises for a suitable use a sponge with a contact surface area structured to provide for contact with a patient's scalp.

The system according to the invention includes a camera 13 to capture a partial image of the patient 10 and a screen to present to said patient 10 a combination of said partial patient's own image and a complementary image provided by an animated digital image representative of a movement of a body injured part of the patient, in such a way the combined image is capable of generating a visual illusion suitable to correct mismatching between motor commands and sensory feedback, said combination of images being prepared or generated under control of said computer device 12 and dedicated software running on it.

The screen 14 can be provided by the own computer device 12 or by an auxiliary screen as in the Fig. 1.

The computer device 12 include a net connection that provide in operation a connection to a remote medical centre that can evaluate o supervise any of said patients sessions and provide to said patient complementary operating instructions.

As to the control interface 15 it comprises an electronic circuit with a wireless communication unit allowing a reliable communication with said computer device 12

In a preferred embodiment both of the control interface 15 and the power supply are associated to the cap 11. For example the power supply comprises one or more batteries attached to the cap and the control interface 15 can also be attached to the cap.

As detailed in Fig. 2 the control interface 15 of the tDCS comprises means for a checking of:
- impedance (Ω) of each of the connecting sections from each of the electrodes to the power source; and
- polarity of each of the electrodes;
acting as a security measure to confirm right positioning of the electrodes and feed current provided to them.

The control interface 15 of the tDCS further includes means for further managing:
- duration and conditions of feeding of said electrical current that is a DC current (the selected current level is provided through DC pulses under a width control);
- control of the digital image on the screen; and
- capture of the person's image or part thereof.

According to a preferred implementation of the system and method of this invention the selected current level is initially provided according to an up current ramp having a selected duration and current application is terminated according to a down current ramp having a selected duration.

## Claims

1. System for neuropathic pain rehabilitation, including:
a transcranial direct current stimulation (tDCS) apparatus comprising:
- a cap(11) sized to fit over a portion of a patient's head;
- two or more connecting points for electrodes operatively associated with an inner surface of the cap (11); and
- a power supply in electrical communication with the electrodes and with a control interface (15) both said power supply and said control interface (15) being sized to be worn by and transported by a patient (10) in use;
**characterized in that** it further comprises:
a computer device (12) with a dedicated software and an associated control unit to provide a patient's self operation of said tDCS apparatus acting through said control interface (15) during a neuropathic pain rehabilitation session initiated by said patient.;
at least a camera (13) to capture at least a partial image of said patient (10);
a screen (14) to present to said patient (10) a combination of said partial patient's own image and a complementary image provided by an animated digital image representative of a movement of a body injured part of the patient (10) capable of generating a visual illusion suitable to correct mismatching between motor commands and sensory feedback, said combination of images being prepared or generated under control of said computer device (12) and dedicated software; and
means for a checking of:
• impedance of each of the connecting sections from each of the electrodes to the power source; and
• polarity of each of the electrodes;
said means for checking acting as a security measure to confirm right positioning of the electrodes and feed current.

2. System, according to claim 1, **characterized in that** said computer device (12) comprises a net connection to a remote medical centre that can evaluate o supervise any of said patients sessions and provide to said patient (10) complementary operating instructions.

3. System according to claim 1, wherein said control interface (15) including an electronic circuit with a wireless communication unit allowing at least a communication with said computer device (12).

4. System according to claim 1, wherein both of said control interface (15) and said power supply are associated to said cap (11).

5. System according to claim 1, wherein each of said electrodes comprises a non invasive electrode pad with and attachment member providing for the selective placement of the electrode at more than one possible position on the inner surface of the cap (11) where a complementary attachment member and electrical connection exists.

6. System according to claim 1 wherein said control interface (15) of the tDCS includes means for further managing:
• duration and conditions of feeding of said electrical current that is a DC current;
• projection on the screen (14); and
• capture of the person's image or part thereof.

7. System according to claim 6 wherein the selected current level is provided through DC pulses under a width control.

8. System according to claim 6 wherein the selected current level is initially provided according to an up current ramp having a selected duration and current application is terminated according to a down current ramp having a selected duration.

9. System according to claim 1, wherein each of said electrodes comprises a sponge with a contact surface area structured to provide for contact with a patient's scalp.

10. System ac cording to claim 4 wherein said power supply comprises one or more batteries attached to the cap (11).

11. System according to claim 1, wherein said control interface (15) is attached to the cap (11).

12. System according to claim 1, wherein said screen (14) is provided by said computer device (12) or by an auxiliary screen.

## Patentansprüche

1. System zur Rehabilitierung neuropathischer Schmerzen, aufweisend:
eine Vorrichtung für transkranielle Gleichstromstimulation (tDCS), umfassend:
- einen Helm (11), welcher dazu dimensioniert ist, auf einen Teil des Kopfes eines Patienten aufgesetzt zu werden;
- zwei oder mehr Anschlusspunkte für Elektroden, welche mit einer inneren Fläche des Helms (11) operativ verbunden sind; und
- eine Energieversorgung in elektrischer Verbindung mit den Elektroden und mit einer Steuerschnittstelle (15), wobei sowohl die genannte Energieversorgung als auch die genannte Steuerschnittstelle (15) dazu dimensioniert sind, bei Verwendung, von einem Patienten (10) getragen und transportiert zu werden;
**dadurch gekennzeichnet, dass** es Folgendes umfasst:
eine Computervorrichtung (12) mit einer bestimmten Software und einer zugehörigen Steuereinheit, um den Selbstbetrieb der genannten tDCS-Vorrichtung seitens eines Patienten zu ermöglichen, unter Benutzung der genannten Steuerschnittstelle (15) während einer Sitzung zur Rehabilitierung neuropathischer Schmerzen, die von dem genannten Patienten begonnen wird; zumindest eine Kamera (13), um zumindest ein Teilbild des genannten Patienten (10) zu erfassen;
einen Bildschirm (14), um dem genannten Patienten (10) eine Kombination aus dem genannten Teilbild des eigenen Patienten und einem komplementären Bild vorzustellen, das von einem animierten Digitalbild bereitgestellt wird, welches eine Bewegung eines verletzten Körperteils des Patienten (10) darstellt, wobei die Kombination eine optische Vorstellung erzeugen kann, die dazu geeignet ist, die fehlende Übereinstimmung zwischen den motorischen Befehlen und der sensorischen Rückmeldung zu korrigieren, wobei die genannte Kombination aus Bildern unter Kontrolle der genannten Computervorrichtung (12) und der genannten bestimmten Software vorbereitet oder erzeugt wird; und
Mittel zur Überprüfung von:
• der Impedanz von jedem der Anschlussabschnitten von jedem der Elektroden zur Energiequelle; und
• der Polarität von jedem der Elektroden;
wobei die genannten Mittel zur Überprüfung als Sicherheitsmaßnahme wirken, um die richtige Positionierung der Elektroden und den Zuführungsstrom zu bestätigen.

2. System nach Anspruch 1, **dadurch gekennzeichnet, dass** die genannte Computervorrichtung (12) eine Netzverbindung zu einem entfernten medizinischen Zentrum umfasst, welches die genannten Sitzungen des Patienten auswerten oder überwachen und dem genannten Patienten (10) komplementäre Arbeitsanweisungen geben kann.

3. System nach Anspruch 1, wobei die genannte Steuerschnittstelle (15) eine elektronische Schaltung mit einer Einheit für drahtlose Kommunikation aufweist, welche zumindest eine Kommunikation mit der genannten Computervorrichtung (12) ermöglicht.

4. System nach Anspruch 1, wobei sowohl die genannte Steuerschnittstelle (15) als auch die genannte Energieversorgung mit dem genannten Helm (11) verbunden sind.

5. System nach Anspruch 1, wobei jede der genannten Elektroden ein nicht invasives Elektrodenpad mit einem Befestigungselement umfasst, welches die selektive Anbringung der Elektrode auf mehr als eine mögliche Position auf der inneren Oberfläche des Helms (11) ermöglicht, auf der es ein komplementäres Befestigungselement und eine elektrische Verbindung gibt.

6. System nach Anspruch 1, wobei die genannte Steuerschnittstelle (15) der tDCS Mittel umfasst, für die zusätzliche Regelung von:
• der Dauer und der Bedingungen für die Zuführung von dem genannten elektrischen Strom, der ein Gleichstrom ist;
• der Projektion auf dem Bildschirm (14); und
• der Erfassung von dem Bild der Person oder von einem Teil desselben.

7. System nach Anspruch 6, wobei der ausgewählte Strompegel durch Gleichstrompulse mit Kontrolle der Breite bereitgestellt wird.

8. System nach Anspruch 6, wobei der ausgewählte Strompegel zunächst gemäß einer Aufwärts-Stromrampe, die eine ausgewählte Dauer aufweist, bereitgestellt wird und die Stromanwendung gemäß einer Abwärts-Stromrampe, die eine ausgewählte Dauer aufweist, beendet wird.

9. System nach Anspruch 1, wobei jede der genannten Elektroden einen Schwamm mit einer Kontaktfläche aufweist, die strukturiert ist, um einen Kontakt mit der Kopfhaut eines Patienten bereitzustellen.

10. System nach Anspruch 4, wobei die genannte Energieversorgung eine oder mehrere Batterien umfasst, die an dem Helm (11) befestigt sind.

11. System nach Anspruch 1, wobei die genannte Steuerschnittstelle (15) an dem Helm (11) befestigt ist.

12. System nach Anspruch 1, wobei der genannte Bildschirm (14) von der genannten Computervorrichtung (12) oder von einem Hilfsbildschirm bereitgestellt wird.

## Revendications

1. Système pour la réhabilitation en douleur névropathique comprenant:
un appareil de stimulation transcrânienne de courant continu (tDCS) comportant:
- un casque (11) ayant la taille approprié pour s'accoupler à une partie de la tête du patient;
- deux ou plusieurs points de connexion pour les relier opérationnellement à une surface intérieure du casque (11); et
- une alimentation en énergie en communication électrique avec les électrodes et ayant une interface de contrôle (15), aussi bien l'alimentation d'énergie que cette interface de contrôle (15) ayant une taille permettant d'être portées et transportées par un patient (10) pendant son utilisation;
**caractérisé en ce qu'**il comporte en plus:
un dispositif informatique (12) ayant un logiciel dédié et un élément de contrôle associé pour permettre au patient de faire fonctionner lui-même cet appareil tDCS en agissant par le biais de cette interface de contrôle (15) durant une séance de réhabilitation en douleur névropathique entamée par ce patient;
au moins une caméra (13) pour capter, au moins en partie, une image de ce patient (10);
un écran (14) pour présenter à ce patient (10) une combinaison de cette image en partie du propre patient et une image complémentaire fournie par une image numérisée animée représentant un mouvement de la partie blessée du corps du patient (10) capable de générer une illusion visuelle appropriée pour corriger le décalage entre les commandes motrices et la rétroaction sensorielle, cette combinaison d'images étant préparée ou générée sous le contrôle de ce dispositif informatique (12) et logiciel dédié ; et
des moyens pour vérifier:
• l'impédance de chacune des sections de connexion de chacune des électrodes à l'alimentation en énergie; et
• la polarité de chacune des électrodes;
ce moyen pour vérifier agit comme mesure de sécurité pour confirmer le positionnement correct des électrodes et du courant d'alimentation.

2. Système, conformément à la revendication 1, **caractérisé en ce que** ce dispositif informatique (12) comporte une connexion au réseau jusqu'à un établissement médical éloigné pouvant évaluer ou superviser toute séance des patients et donner des instructions de fonctionnement complémentaires à ce patient (10).

3. Système conformément à la revendication 1, dans lequel cette interface de contrôle (15) comporte un circuit électronique ayant un élément de communication sans fil permettant au moins une communication avec ce dispositif informatique (12).

4. Système conformément à la revendication 1, dans lequel aussi bien l'interface de contrôle (15) que cette alimentation en énergie sont reliés à ce casque (11).

5. Système conformément à la revendication 1, dans lequel ces électrodes comprennent une plaquette d'électrode non-invasive ayant un membre de fixation pour le placement sélectif de l'électrode à plus d'une position possible sur la surface intérieure du casque (11) là où ce membre de fixation complémentaire et la connexion électrique existent.

6. Système conformément à la revendication 1 dans lequel cette interface de contrôle (15) du tDCS comporte des moyens pour gérer en plus:
• la durée et les conditions d'alimentation de ce courant électrique qui est un courant continu.
• la projection sur l'écran (14); et
• la captation de l'image de la personne ou une partie de celle-ci.

7. Système conformément à la revendication 6 dans lequel le niveau de courant sélectionné est fourni par le biais d'impulsions de courant continu sous un contrôle de largeur.

8. Système conformément à la revendication 6 dans lequel le niveau de courant sélectionné est initialement fourni conformément à une rampe de courant ascendant ayant une durée sélectionnée et l'application du courant est finie conformément à une rampe de courant descendant ayant une durée sélectionnée.

9. Système conformément à la revendication 1, dans lequel chacune de ces électrodes comporte une éponge ayant une région de surface de contact structurée pour fournir le contact au cuir chevelu d'un patient.

10. Système conformément à la revendication 4 dans lequel cette alimentation en énergie comporte une ou plusieurs batteries reliées au cuir chevelu(11).

11. Système conformément à la revendication 1 dans lequel cette interface de contrôle (15) est fixée sur le casque (11).

12. Système conformément à la revendication 1 dans lequel cet écran (14) est pourvu de ce dispositif informatique (12) ou d'un écran auxiliaire.
